# EUROPEAN PATENT APPLICATION

(11) **EP 3 456 343 A1**
(43) Date of publication of application: **20.03.2019**
(21) Application number: 17185596.8
(22) Date of filing: 09.08.2017
(51) Int. Cl.: A61K 39/00, A01N 1/02, A61P 35/00

(54) **VACCINE FORMULATION OF DENDRITIC CELLS**

(71) Applicant: Dodge Private Equity Ltd., Charlestown (KN)
(72) Inventor: IMHOF, Marianne, 2512 Tribuswinkel (AT)

(57) **Abstract**

The present invention relates to cryo-conserved formulations of dendritic cells free of serum components and to ready-to-use formulations of said dendritic cell, specifically to the production of vaccines containing processed dendritic cells.

## Description

### Field of the Invention

The present invention relates to the field of vaccine formulation, specifically to formulations of vaccines comprising processed dendritic cells.

### Background Art

The broad use of autologous cancer vaccines, including dendritic cells or whole tumor cells, is limited by both the availability of patient's samples or specimens and the complex procedure of preparing individualized vaccines (M. Lluesma et al. Biomedicines. 2016 May 3;4(2)).

The common setting to prepare cell vaccines for administration resembles the following: collected DCs were washed five times with normal saline, and 1.5 × 10⁶ DCs were suspended in 0.3 mL normal saline supplemented with 1 % heat-inactivated autologous plasma. To remove cell clusters, the cell suspension was slowly passed through a 25-gauge needle. The cell suspension was then injected subcutaneously near one inguinal lymph node of the patient (Ko-Jiunn Liu et al., Journal of Biomedical Science 2016 23:64).

WO0216560 describes a method for producing ready to use, antigen loaded or unloaded, cryo-conserved mature dendritic cells for the production of a vaccine containing said dendritic cells. The dendritic cells are frozen in freezing medium containing autologous serum, DMSO and glucose.

This or similar procedures might be suitable for clinical trials. However, in everyday busy clinical and outpatient settings this approach inherits significant caveats, which may lead to diminished sterility and efficacy of live cell vaccines. Repeated cleaning steps after thawing of the limited pulsed autologous cells, already in a very sensitive status due to the procedure of thawing, washing steps including centrifugation and temperature variances should be ideally performed as fast as possible. This does not seem realistic and probably the procedure results in significant reduction in cell numbers of active dendritic cells to be administered.

There is still a need for a ready-to-use formulation of dendritic cells, which is applicable for routine administration with a maximum of viability of the dendritic cells and can easily be applied intradermal.

### Summary of invention

It is an objective of the present invention to improve cell performance by an approach of highest possible parsimony during preparation of cell vaccines. The object is solved by the subject matter of the present invention.

The present invention relates to a pharmaceutical composition of dendritic cells, wherein the dendritic cells are frozen in a freezing medium consisting of 5-25 (v/v)% DMSO and physiological water solution of 2.5 to 10 (w/v)% glucose. Specifically the dendritic cells are processed dendritic cells, preferably loaded dendritic cells, more preferably double loaded dendritic cells.

In some embodiments of the invention the pharmaceutical comprised dendritic cells in a concentration of 1x10⁶ to 1x10⁸ cells/mL, or in a concentration of 1x10⁷ to 10x10⁷.

In some embodiments of the invention the pharmaceutical composition comprised dendritic cells which are loaded with at least one nucleic specific acid molecule encoding a tumor associated antigen or fragment thereof and at least one protein molecule being a tumor associated antigen or fragment thereof. Specifically, the dendritic cells are loaded with TERT-mRNA and survivin peptide.

A further embodiment of the invention relates to a method for the preparation of ready-for-use cryo-conserved double loaded dendritic cells, the method comprising:
- providing immature dendritic cells;
- loading said cells with at least one nucleic specific acid molecule encoding a tumor associated antigen or fragment thereof and at least one protein molecule being a tumor associated antigen or fragment thereof,
- culturing the immature dendritic cells in a culture medium containing a maturing cocktail comprising one or more maturing stimulants to obtain mature dendritic cells;
- freezing the mature dendritic cells in a freezing medium consisting of 5-25 (v/v)% DMSO and physiological water solution of 2.5 to 10 (w/v)% glucose.

In some embodiments of the invention, the nucleic specific acid molecule is TERT-mRNA and the protein molecule is survivin, or fragments thereof.

A further embodiment of the invention relates to a vaccine comprising the pharmaceutical composition of any one claim 1 to 4 and a sterile saline solution.

In some embodiments of the invention the vaccine as described herein comprising about 1x10⁶ to 1x10⁸, or in a concentration of 1x10⁷ to 10x10⁷ double loaded dendritic cells/mL, 5-25 % DMSO, physiological water solution of 2.5 to 10 % glucose, and 0.5 to 1.5 % sterile saline solution.

In some embodiments of the invention, the dendritic cells of the vaccine as described herein are loaded with TERT-mRNA and survivin peptide.

A further embodiment of the invention relates to a vaccine as described herein for use in the treatment or prevention of cancer.

In some embodiments of the invention the vaccine is administered intradermal. Specifically, the vaccine is administered by way of intradermal depots, e.g., 1, 2, 3, 4, 5, or 6 intradermal depots are generated.

A further embodiment of the invention relates to a method for the preparation of the vaccine of any one of claims 7 to 9, the method comprising:
- thawing the frozen pharmaceutical composition of any one of claims 1 to 4, and
- adding sterile saline solution to the thawed solution of step a).

A further embodiment of the invention relates to a vaccination kit or set for vaccination, comprising a vial comprising the pharmaceutical composition as described herein, a second vial comprising sterile saline solution and a package insert with administration instructions, wherein the vaccination kit or set is operatively assembled to perform the administration of the vaccine to a patient and to elicit a safe and protective immune response against the antigen. Specifically, the antigens are TERT and survivin.

### Brief description of drawings

Fig. 1 shows the results of 4 different processing procedures in living cell numbers.
Fig. 2 depicts vaccine specific immune responses.
Fig. 3 depicts TERT- (above 2 panels A and B) and survivin-specific (lower 2 panels C and D) IFNγ release cell frequencies are shown (y-axis). On the left side results for CD8+ T-cells and on the right side results for CD4+ cells are displayed. *P-*values are retrieved by Wilcoxon Signed-Rank Test. X-axis: pre = pre-vaccination, 3^{rd} = after 3^{rd} vaccination
Fig. 4 depicts PFS in months of Procure® patients. Horizontal bars show progression free survival of 14 study patients (patient ID on the y-axis, * progression, ° no contact, > still in remission) in months (x-axis). The dashed vertical line at 15.9 months indicates the median of the matched historical control, the solid black vertical line indicates the median at 46.7 months of Procure patients.

### Description of embodiments

The present invention relates to a ready-to-use formulation of dendritic cells, preferably to dendritic cells loaded with at least one nucleic acid molecule encoding a tumor associated antigen or fragment thereof and at least one proteic molecule being a tumor associated antigen or fragment thereof and methods for manufacturing the same.

Dendritic cells (DCs) are bone-marrow-derived antigen-presenting cells (APCs) that play a pivotal role in both induction and regulation of the immune response. It has been described that the manipulation of DCs, in order to produce a natural vaccine adjuvant can be particularly effective in stimulating anti-tumor immunity. In humans, diminished DCs function has been documented in patients with several different tumor types (breast, head and neck, colorectal and renal cell cancer, hepatocellular carcinoma and malignant melanoma). These changes have been seen mainly in blood, in cells infiltrating cancers and more recently in lymphonodes (LNs). All the studies have confirmed the defective DC function in the blood of patients as well as the substantial inhibition of DC activity in both blood and tumor-draining LNs. Moreover, it has been documented that most chemotherapies deeply impair DC function, whereas the responses of the T cells from patients are comparable with those of healthy donors, suggesting that the defects in the functional activity of DCs constitute the major factor in the inability of the host immunosystem to elicit an appropriate and beneficial anticancer immune response. The defects in the functionality of cancer patients'DCs correlate as well with an impaired production of IL-12. In addition, it was shown that the IL-10 levels in serum from patients with cancer are significantly higher than those found in normal age- and sex-matched controls. IL-10 has been shown to have a significant inhibitory effect on several aspects of DC function, such as the expression of costimulatory molecules and the ability to synthesize IL-12. Importantly, IL-10-treated DCs become tolerogenic. Moreover, in patients with progressively tumor growing, the DCs are both immature and switched off. Since the antitumor effect of DCs is dependent on their level of activation and maturation, the lack of costimulatory molecules, associated with hindered maturation, is likely to result in failure of induction of a tumor-specific T cell cytotoxic response.

The most common approach to using DCs for vaccines is to prepare large numbers of autologous mature myeloid DC (MDCs) ex vivo, load them with antigens, and inject them back into the subject.

The double loading with a nucleic acid molecule and proteic tumor antigen ensures both a short-term response of the immune system by activating the cytotoxicity through T-cytotoxic, NK and NKT as well as a long-term response through the activation of the T-mem. In this way, the reiteration of DCs injections is not anymore required on the long period.

The DCs as described in WO2009021982 thus loaded with two different types of molecules: a nucleic acid molecule and a proteinaceous molecule.

According to a preferred embodiment the tumor associated antigen or fragment thereof encoded by the at least one nucleic acid molecule is selected from the group consisting of survivin and telomerase reverse transcriptase (TERT).

The DCs are loaded with mRNA-encoding antigen for the following reasons:
- mRNA-transfected DCs are equally or more potent than peptide-pulsed DCs in stimulating CTL responses *in vitro;*
- mRNA-transfected DCs are most effective APCs, capable of stimulating CD8+ responses *in vitro;*
- mRNA transfection is so effective that it can induce and contribute to the DC maturation process; and
- mRNA can be loaded on the DCs via passive transfection with high efficiency, as measured as CTL response.

Although the mRNA and nucleic acid molecule loading method shows several advantages on other loading techniques, this approach has as well an Achilles' heel: although it triggers a potent CTL response through the activation of CD8⁺ cells, it cannot induce a CD4⁺ response, even if such a response has been shown critically for the tumor immunity. Indeed, CD4⁺ cells provide important functions for the expansion and persistence of CD8⁺ CTLs, stimulate the innate arm of the immune system at the tumor site, and inhibit local angiogenesis. An optimal anti-tumor immune response therefore requires the concomitant activation of both the CD8⁺ and the CD4⁺ T-cell arms of the immune response.

In order to achieve this goal, a second loading step, along with mRNA loading approach is introduced: proteic antigen loading by passive incubation. DCs stimulated by mRNA incubation, show an increased capability, for example, to phagocyte short peptides. Therefore, it turned out that the use of the double-loading approach in order to induce both CD8⁺ and CD4⁺ T-cell arms of the immune response is advantageous. This approach constitutes a great improvement of the DCs-based cancer immune therapy, since it can, at the same time, induce the short-time response by activation of the CD8⁺ and the long-lasting memory response, through the activation of the CD4⁺ T-cell.

Thus, the double loading approach has several advantages on other methods, known in the art:
- specific activation of both the cytotoxic and the memory arms of the immune system;
- absence of the T-reg response;
- use of different antigens to direct the activation of each of the immune system arm with consequent gain in both sensitivity and strength of the stimulus;
- therapy virtually applicable to any type of solid and blood tumor;
- combination of universal and type-specific tumor antigens;
- total conformity to GMP- and re-vivo use;
- ready-to-use product that can be stored for years;
- minimal cell manipulation;
- no requirement for tumor tissue/cells;
- combination of the positive effects coming from endogenous production of tumor antigen (encoded by the mRNA) and from endocyted proteic antigen;
- high cell viability after a cycle of freezing/thawing;
- total preservation of cell functionality and phenotype after a cycle of freezing/thawing; and
- versatility of the method.

Procure® comprises unique double loaded dendritic cells which have been loaded with the universal tumor antigens survivin and TERT.

However, handling of the vaccine is still cumbersome and challenging. While the investigational medicinal product (IMP) can be stored between -80 °C and -150 °C for months, once sealed in the transport box, the lifespan is shortened to four days. Once the transport box is opened at the clinical site, the product must be immediately be thawed within a maximum of 10 min by rubbing the vial in the hands and be promptly administered.

According to the present invention, the double loaded and matured dendritic cells are frozen in a freezing medium which is free of heterologous and non-heterologous serum components. The freezing medium consists of the cryoprotectant DMSO and a physiological water solution of glucose. Notably, the freezing medium is free of any autologous serum or plasma.

The cryoprotectant DMSO may be present in a concentration of about 5-25 (v/v)%, or of about 7.5-20 (v/v)%, or about 10 (v/v)%.

The physiological water solution may comprise glucose in a concentration of 2.5-10 (w/v)%, or about 5 (w/v)% glucose.

In one embodiment of the invention freezing medium consists of about 10 (v/v)% DMSO and the physiological water solution containing about 5 (w/v)% glucose.

According to the present invention, the dendritic cells are suspended in 0.1 to 1.0 mL of the freezing medium, or in about 2.5 to 7.5 mL, specifically in 0.5 mL.

The dendritic cells are frozen in the freezing medium at a concentration of about 1x10⁶ to 1x10⁸/mL, or in a concentration of about 1x10⁷/mL to 10x10⁷/mL, or on a concentration of about 2.5x10⁷/mL.

A further embodiment of the invention relates to a ready-to-use vaccine, wherein the frozen dendritic cells are thawed and sterile saline solution is added. The ready-to-use vaccine thus consists of dendritic cells, DMSO, physiological water, glucose and sterile saline solution. In some embodiments of the invention the sterile saline solution is in a concentration of about 0.5 to 1.5 (w/v)%, or of about 0.9 (w/v)%.

In clinical tests it has been shown that treatment with PROCURE® could delay or ultimately prevent tumor recurrence, specifically in the treatment of ovarian cancer. Thus, a further embodiment of the invention relates to the vaccine as described herein, for use in the treatment of cancer. Specifically, the invention relates to delaying or preventing tumor recurrence.

The vaccine may be administered parenteral, specifically intradermal. The vaccine may be administered as a depot under the skin of the patients. In some embodiments of the invention the vaccine as administered as 1, 2, 3, 3, 5, or even 6 intradermal depots, specifically as 4 depots.

Due to the physiological formulation, the vaccine is well tolerated by the subjects.

### Examples

The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

### Preclinical testings and results

Preclinical test series have been performed to investigate the viability of double loaded dendritic cells after various thawing procedures. Cells were thawed at 37 °C and then suspended in the following solutions: 10 % DMSO, solved in a physiologic water solution of 5% glucose dilutions 1:4 with 0.9 % NaCl (2.5 % DMSO final concentration) and 1:10 with 0.9 % NaCl (1 % DMSO final concentration) thereof. As comparator cells were washed 3 times in PBS and resuspended in media. Scenario 1, all steps were performed in an optimal sequence with no waiting times, scenario 2 most likely "sitting" times have been included (10 min after centrifugation, 20 min after resuspension in media at room temperature) to simulate daily situations in clinical operations.

Cells were then incubated at 37 °C and 5 % CO₂ in 96-well dishes for 10, 30 and 60 minutes. Viability of cells was determined by trypan blue assay counting cells with Neubauer chamber in 5 quadrants each. All tests were performed with 3 different cell batches in triplicate.

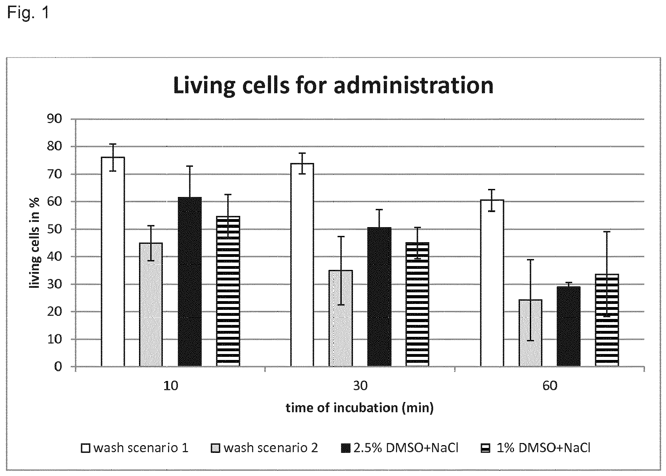

Fig. 1 depicts the results of 4 different processing procedures in living cell numbers. The highest percentage of living cells is after washing and ideal circumstances with mean of 76% (white bars, wash scenario 1). This percentage drops significantly to a mean of 45% when there are some sitting times included in the protocol (see grey bars, wash scenario 2). The tested direct dilution with 0.9% NaCl with 2.5% DMSO final concentration results in the second highest percentage of living cells of 61 % (black bars). Further dilution of DMSO to 1 % does not significantly improve the cell numbers (striped bars).

In the clinical testing PROCURE® consisted of 1.3*10⁷ autologous, fully mature DCs, double loaded with TERT-mRNA and survivin-peptide, diluted in 0.5 mL of a standard freezing solution, made up of 10 % DMSO, solved in a physiologic water solution of 5 % glucose; provided in a 2 mL cryo-vial labeled according to the cGCP requirements. The cell therapy was supplied deep-frozen in dry ice, contained in a standard, temperature-controlled box specifically designed for such a transport. While the IMP can be stored between -80 °C and -150 °C for months, once sealed in the transport box, its lifespan is shortened to four days. Once the transport box has been opened at the clinical site, the product must be immediately thawed within a maximum of 10 min by rubbing the vial in the hands and promptly administered.

### Preparation of the cell suspension and method of administration

As consequence of the results of the preclinical experiments the following procedure has been developed for PROCURE® to maximize viability by dilution and still have a total volume that can be easily applied intradermally (2 mL).

DCs suspended in 0.5 mL solution are delivered in a 2 mL cryovial with a screw cap. After the thawing procedure (rubbing the vials in hands) 1.5 ml of the 0.9% sterile saline solution provided into the 5 mL syringe is drawn up. No needle is needed for this step. The saline vial is connected with the syringe via a Luer lock. Then a sterile single use needle (20 G 0.90 x 40) is attached onto the syringe, the cryovial with the DC vial opened and slowly the saline solution is added to dilute PROCURE® without causing any bubbles. With this step all DCs should be detached from the surface of the vial. Next, the entire resulting 2 mL of dilution of DCs is drawn up - slowly again to minimize damage of the fragile cells. Now needles are exchanged in a sterile manner. An intradermal sterile single use needle (24 G 0.55 x 25) is used to immediately administer the IMP paraumbilically into the skin (intradermal) by setting 4 to 5 wheals (intradermal depots) with an approximate volume of 0.5 mL each. The time between removing the vial from the Bio-Pouch and administration must not exceed 10 min.

### Clinical trial & results

The above introduced preparation of the cell suspensions and method of administration has been followed during a phase I/IIa clinical trial.

The study was an international multicenter phase I/IIa, open labeled, randomized study to compare two intradermal vaccination schedules of an active immunotherapy with fully mature, TERT-mRNA and survivin-peptide double loaded dendritic cells (DC): weekly (group A) versus every two weeks (group B) with trial number NCT01456065. Primary endpoint was safety, and secondary endpoint was quality of life, efficacy analysis in terms of biomarker analysis, immune activation and tumor progression.

Procure® was administered as an intradermal, paraumbilical injection at a dosage of 1.3 x 10⁷ autologous, fully mature DCs, double loaded with TERT-mRNA and survivin-peptide, diluted in 0.5 mL of a standard physiological solution made up of 10 % DMSO, solved in a physiologic water solution of 5 % glucose.

Both treatment arms received up to eight injections, arm A once every week and arm B once every two weeks. There were no adjuvants applied or premedication recommended. The patients were followed for a total of 12 (group A) and 19 (group B) weeks after the first dose, comprising 8 treatment and 1 follow-up visit. The first six patients were hospitalized for treatment till the approval of the interim safety report. The consecutive block of nine patients was treated in an outpatient setting.

Patients were evaluated on a regular basis during every treatment and follow up. Before each vaccination all safety parameters were recorded according to the NCI-CTC criteria (CTC-Version 3.0.). Additionally, full hematology, clinical chemistry and CA125 measurement was performed. Biomarker parameters were assessed in patients before starting the vaccination and before the fourth and seventh Procure-application. Follow-up monitoring was performed at 5 weeks after the last treatment. Out of protocol observations by the investigators concerning progression free survival or death were collected with last data log 27 June 2017.

### Vaccine-specific immune response assessment

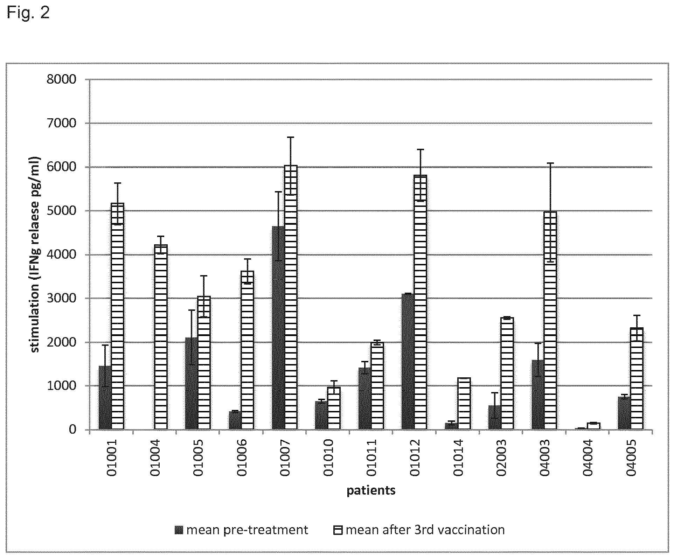

Vaccine-specific immune responses were evaluable in 6 (75.0 %) and 6 (85.7 %) patients in groups A and B, respectively. Patients not included in the statistical analysis: 01013 has been withdrawn after visit 1, for 01004 the pre-treatment data point was missing and for patient 01008 no autologous double loaded dendritic cells for stimulation of the PBMCs were available. For the vaccine-specific cytokine release assay 12/12 evaluable patients met the predefined criterion of 1.2 fold induction from pre-vaccination to after third vaccination. Results exceeded the 1.2 limit and even a 1.25 limit in all cases with a median of 3.1 and mean of 3.6 (min 1.30; max 8.7). These results translate to a 100 % positive immune response to the IMP (Fig. 2) with high significance, *P*=0.002 (Wilcoxon Signed-Rank Test). In addition, this immune response was further increased in 3/12 patients after the sixth vaccination. In general there were no differences between dosing regimens.

As shown in Fig. 2, IFNγ release of PBMCs stimulated with the autologous dendritic cell vaccine Procure® before (black bars) and after third treatment (striped bars). Error bars indicate standard deviations of duplicates. For patient 01004 the pre-vaccination data point is missing.

### Detection of TERT and survivin antigen-specific T-cells

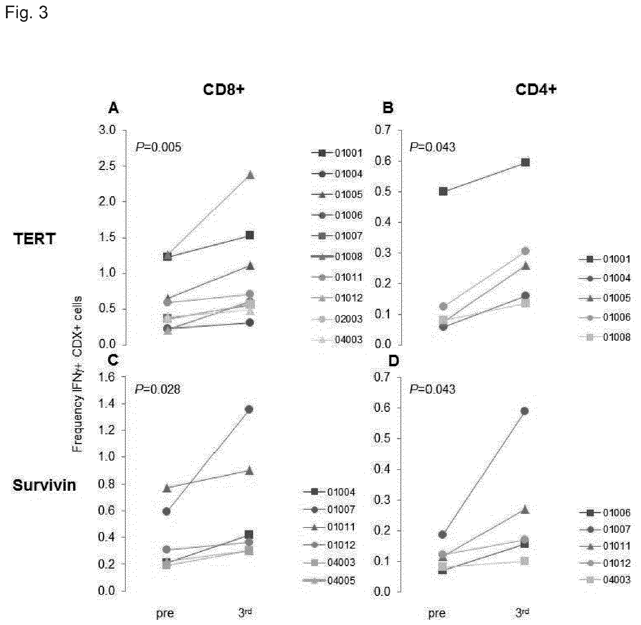

TERT- and survivin-specific cytokine release assays revealed a qualitative antigen-specific immune response for both TERT and survivin in CD4+ as well as CD8+ T-cells after the 3^{rd} vaccination compared to baseline (Fig. 3). TERT CD8+ responses could be analyzed for 14 patients, 10 reached the cut off of 1.2-fold induction with high significance of *P*=0.005 (Fig. 3A). TERT CD4+ responses could be analyzed for 7 patients, 5 reached the cut off of 1.2-fold induction with significance of *P*=0.043 (Fig. 3B). Survivin CD8+ responses could be analyzed for 13 patients, 6 reached the cut off of 1.2-fold induction with significance of *P*=0.028 (Fig. 3C). Survivin CD4+ responses could be analyzed for 12 patients, 5 reached the cut off of 1.2-fold induction with significance of *P*=0.043 (Fig. 3D). All significance levels were derived with the same test statistics by Wilcoxon Signed-Rank Test.

### Clinical efficacy

The majority of patients (14/15) did not have tumor progression between baseline and follow-up (week 12/19). There was also no change from baseline overall in CA-125 for either dosing regimen. All patients in the analysis population had negative computed tomography (CT) scans at all scheduled visits. Patient 01013 had documented tumor progression at visit 1 (week 0), which was present prior to the administration of IMP. Patient 04004 had tumor progression at the week 48 follow-up according to first protocol version, which was 238 days after the last administration of IMP. Neither of the tumor progressions was considered to be related to the IMP.

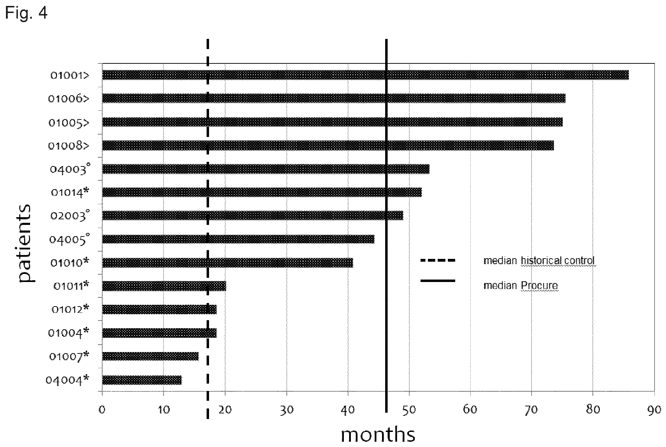

Quaterly updated out of protocol follow-up observations of the investigators with last data log 27 June 2017 exhibited a median progression free survival (PFS) from start of chemotherapy of >46 months (Fig. 4). Data were compared to matched historical control data (FIGOIII, residual tumor size 0mm, 1-10mm, >10mm) from the literature (duBois et al. 2008). Procure PFS data did not correlate with residual tumor size (R²=0.0291).

Fig. 4 depicts out of protocol progression free survival data versus matched historical control. Horizontal bars show progression free survival of 14 study patients (patient ID on the y-axis, * progression, ° no contact, > still in remission) in months (x-axis). The dashed vertical line at 15.9 months indicates the median of the matched historical control, the solid black vertical line indicates the median at 46.7 months of Procure® treated patients.

## Claims

1. A pharmaceutical composition of double loaded dendritic cells, wherein the double loaded dendritic cells are frozen in a freezing medium consisting of 5-25 (v/v)% DMSO and physiological water solution of 2.5 to 10 (w/v)% glucose.

2. The pharmaceutical composition of claim 1, wherein said cells are in a concentration of 1x10⁶ to 1x10⁸ cells/mL, or in a concentration of 1x10⁷ to 10x10⁷.

3. The pharmaceutical composition of claim 1 or 2, wherein said cells are loaded with at least one nucleic specific acid molecule encoding a tumor associated antigen or fragment thereof and at least one protein molecule being a tumor associated antigen or fragment thereof.

4. The pharmaceutical composition of any one of claims 1 or 3, wherein the dendritic cells are loaded with TERT-mRNA and survivin peptide.

5. A method for the preparation of ready-for-use cryo-conserved double loaded dendritic cells, the method comprising:
a. providing immature dendritic cells;
b. loading said cells with at least one nucleic specific acid molecule encoding a tumor associated antigen or fragment thereof and at least one protein molecule being a tumor associated antigen or fragment thereof,
c. culturing the immature dendritic cells in a culture medium containing a maturing cocktail comprising one or more maturing stimulants to obtain mature dendritic cells;
d. freezing the mature dendritic cells in a freezing medium consisting of 5-25 (v/v)% DMSO and physiological water solution of 2.5 to 10 (w/v)% glucose.

6. The method of claim 5, wherein the nucleic specific acid molecule is TERT-mRNA and the protein molecule is survivin, or fragments thereof.

7. A vaccine comprising the pharmaceutical composition of any one claim 1 to 4 and a sterile saline solution.

8. The vaccine of claim 7 comprising about 1x10⁶ to 1x10⁸, or in a concentration of 1x10⁷ to 10x10⁷ double loaded dendritic cells/mL, 5-25 % DMSO, physiological water solution of 2.5 to 10 % glucose, and 0.5 to 1.5 % sterile saline solution.

9. The vaccine of claim 7 or 8, wherein said cells are loaded with TERT-mRNA and survivin peptide.

10. The vaccine of any one of claims 7 to 9 for use in the treatment or prevention of cancer.

11. The vaccine for use according to claim 10, wherein the vaccine is administered intradermal.

12. The vaccine for use according to claim 11, wherein at 1, 2, 3, 4, or 5 intradermal depots are generated.

13. A method for the preparation of the vaccine of any one of claims 7 to 9, the method comprising:
a. thawing the frozen pharmaceutical composition of any one of claims 1 to 4, and
b. adding sterile saline solution to the thawed solution of step a).

14. A vaccination kit or set for vaccination, comprising a vial comprising the pharmaceutical composition of any one of claim 1 to 4, a second vial comprising sterile saline solution and a package insert with administration instructions, wherein the vaccination kit or set is operatively assembled to perform the administration of the vaccine to a patient and to elicit a safe and protective immune response against the antigen.

15. The vaccination kit or set for vaccination of claim 14, wherein said antigens are TERT and survivin.
